# EUROPEAN PATENT SPECIFICATION

(11) **EP 4 065 909 B1**
(45) Date of publication and mention of the grant of the patent: **06.11.2024**
(21) Application number: 21718597.4
(22) Date of filing: 19.04.2021
(51) Int. Cl.: F25D 27/00, A61L 2/20, F25D 29/00

(54) **MANUFACTURER OF ICE FOR FOOD USE WITH AN INCORPORATED ICE CONTAINER HAVING AN INTEGRATED SANITISING SYSTEM**
EISBEREITER FÜR LEBENSMITTEL MIT INTEGRIERTEM EISBEHÄLTER MIT INTEGRIERTER ENTKEIMUNGSANLAGE
MACHINE DE FABRICATION DE GLACE À USAGE ALIMENTAIRE COMPORTANT UN RÉCIPIENT À GLACE INCORPORÉ AYANT UN SYSTÈME D'ASSAINISSEMENT INTÉGRÉ

(30) Priority: 06.07.2020 IT 202000016333
(43) Date of publication of application: 05.10.2022
(73) Proprietor: Scotsman Ice S.r.l., 20010 Pogliano Milanese (MI) (IT)
(72) Inventor: VANIA, Tommaso, 20090 Monza (Monza e Brianza) (IT)
(74) Representative: Rapisardi, Mariacristina
(86) International application number: PCT/EP2021/060034
(87) International publication number: WO 2022/008113

(56) References cited:
- WO-A1-03/038351
- DE-U1- 29 722 227
- US-A1- 2005 089 458

## Description

The present invention refers to a fully-automated monobloc machine for manufacturing ice for food use with an incorporated ice container having an integrated sanitising system.

For some time fully-automated monobloc machines have been present on the market, for automated production of ice for food use for internal use by operators in the foodstuffs sector such as hotels, restaurants, catering, food sales and preparation establishment, fishmongers, bars and markets.

It is known that these machines work on a normal refrigeration cycle, where the evaporator is provided with forming means for forming ice particles from liquid drinking water when appropriately sprayed or conveyed onto the means where the drinking water changes from liquid to solid state.

In general machines used for automated production of ice are directly connected to the tubing coming from the municipal water supply with an absence of refining systems of the drinking water used.

Some machines can be provided with a water storage tank: in this case, i.e. when the drinking water used for the ice production is not used in a continuous flow, the automated production of the ice must include further water disinfection systems with disinfectants suitable for the purpose of preventing issues with microbial proliferation.

The ice particles thus formed, such as ice cubes (full or hollow), in flakes or granules, are then detached from the forming means, typically with a brief inversion of the refrigeration cycle, and immediately collected in containers or trays made of suitable materials for the contact with foodstuffs that need being kept clean and sanitised.

The user then manually accesses the container to collect the ice particles, according to needs, while duly following the correct hygiene practices for handling foodstuffs (hand hygiene, clothing hygiene and so on) with the purpose of preventing microbiological and particle contaminations.

As is known, these machines are provided with control means which subordinates the formation of the particles, and thus the functioning of the machine under production, to a minimum level of particles in the collection container.

As is also known, manual access by the user and/or a plurality of users not particularly protected to the collection container can transmit a possible contamination of micro-organisms to the ice particles contained in the collection container, and which will be subsequently collected.

In the present historical moment, special and due attention also needs being paid to the drinking water in the liquid state used for the formation of the ice particles, which also must be sanitised to be freed from micro-organisms.

Systems for sanitising drinking water in machines provided with a water storage tank and based on the disinfection of the water with suitable disinfectants are notoriously poorly effective, as these activities are typically not automatic.

It is well known that these traditional sanitising systems require the user's diligence, precision and goodwill.

The traditional systems for sanitising the collection containers and/or trays for the ice particles produced traditionally include cleaning and sanitising the container and/or the tray, but not sanitising the ice particles between one withdrawal by a user and the next. DE 297 22 227 U1 discloses a fully-automated monobloc machine according to the preamble of claim 1.

US 2005/089458 A1 discloses a further machine of the prior art.

There is therefore a need to simplify the structure of the sanitising systems in a fully-automated monobloc machine for manufacturing ice of known type.

The technical task of the present invention is, therefore, to provide a fully-automated monobloc machine for manufacturing ice in particles for food use which enables obviating the technical disadvantages comprised in the prior art.

Within the context of this technical task an object of the invention is to realise a fully-automated monobloc machine for manufacturing ice in particles for food use which has a system for sanitising the ice particles in the collection container.

A further aim of the invention is to realise a fully-automated monobloc machine for manufacturing ice in particles for food use which has a system for sanitising the drinking water in the storage tank.

A further aim of the invention is to realise a fully-automated monobloc machine for manufacturing ice in particles for food use which has a system for sanitising the ice particles in the collection container and a system for sanitising the drinking water in the storage tank that are simple, effective and have an automated functioning.

The technical task, as well as these and other objects according to the present invention are achieved by realising a fully-automated monobloc machine for manufacturing ice in particles for food use, as defined in claim 1, comprising at least one storage tank of process water in the liquid state, an evaporator provided with forming means for forming ice particles from liquid water, a collection container of the ice particles, an access hatch door to the collection container, wherein it comprises surface sterilisation means of the ice particles in the collection container, detection means of the open or closed position of the access hatch door, and control means communicating with said detection means and configured to activate said sterilisation means if said closed position is detected.

Other characteristics of the present invention are further defined in the following claims.

Further characteristics and advantages of the invention will become more apparent from the description of a preferred, but not exclusive, embodiment of a fully-automated monobloc machine for manufacturing ice in particles for food use according to the invention, illustrated by way of non-limiting example in the appended drawing, in which:

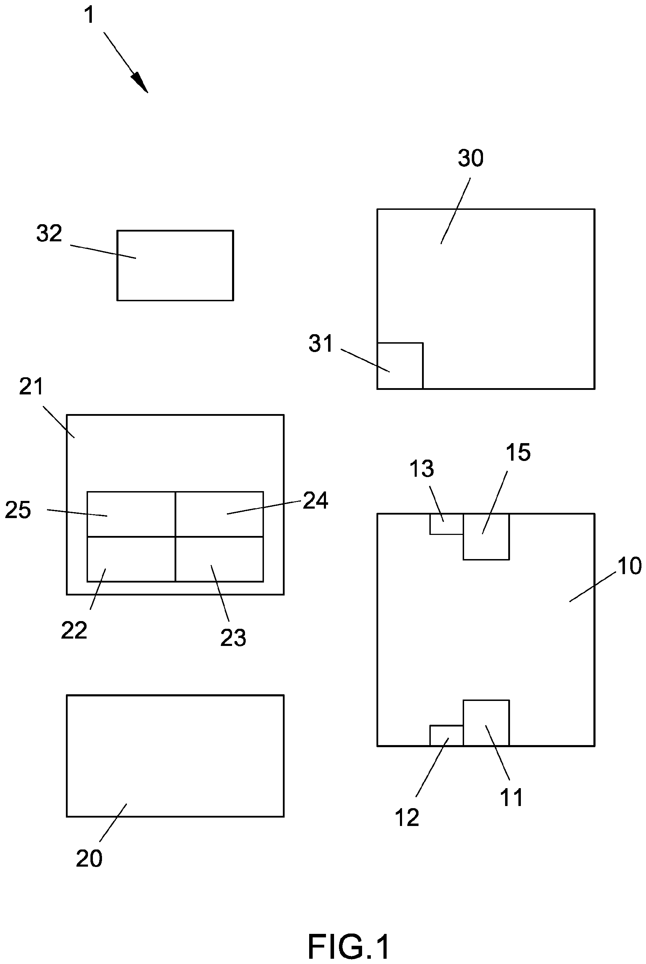

figure 1 is an overall diagram of a fully-automated monobloc machine for manufacturing ice according to the invention;

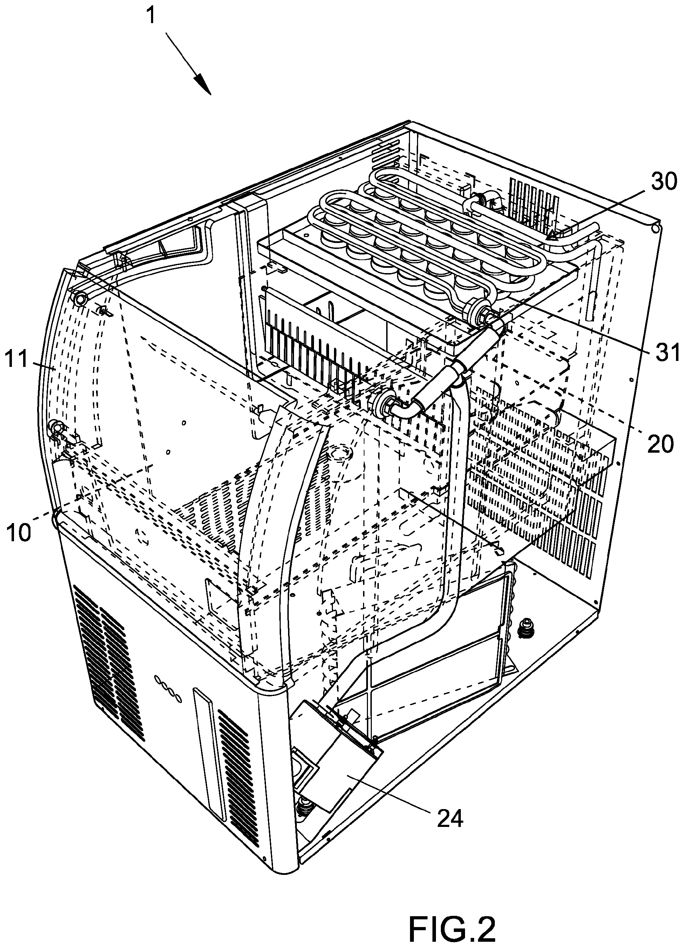

figure 2 is a transparent view of a fully-automated monobloc machine for manufacturing ice according to the invention.

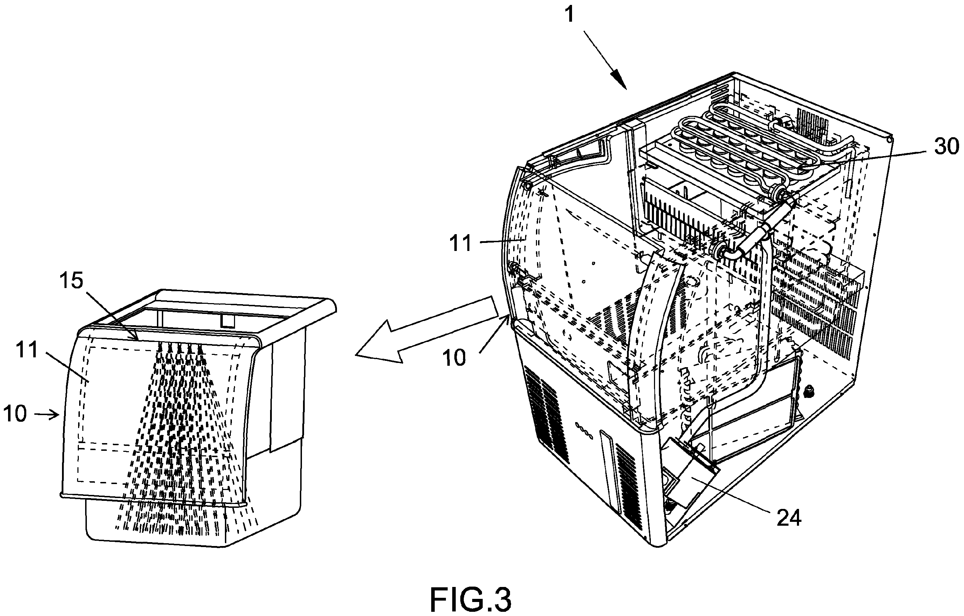

figure 3 is a transparent view of a fully-automated monobloc machine for manufacturing ice with highlighting of a collection container of the ice particles. With reference to the figures mentioned, fully-automated monobloc machine is illustrated for manufacturing ice in particles for food use, denoted in its entirety by reference number 1.

The operation in production of the fully-automated monobloc machine 1 is with a known refrigeration cycle, the components of which are not highlighted in the figure but of which only the evaporator 30 is illustrated provided with forming means for forming ice particles 31 from the process drinking water in the liquid state which is advantageously sprayed or in any case conveyed by operating means 32 onto the forming means 31, where the liquid changes from liquid to solid state. The machine 1 comprises a storage tank 20 where the process drinking water in the liquid state coming from the municipal water supply is stored and subsequently withdrawn by the operating means 32 for the subsequent transformation thereof into ice.

The ice particles thus formed, being ice cubes (full or hollow), in flakes or in granules, are then detached from the forming means 31, typically with a brief inversion of the refrigeration cycle, and immediately generally collected by force of gravity in at least a collection container 10.

According to the present invention, the collection container 10 is advantageously closed, by at least one access hatch door 11.

The user manually accesses the collection container 10 to collect the ice particles, according to needs, exclusively through the access hatch 11, which is normally in the closed position.

According to the present invention, there is a detection means 12 of the open or closed position of the access hatch door 11 present.

The detection means 12 typically comprises a microswitch activated by the opening or closing movement of the hatch door 11.

According to the present invention, the collection container 10 advantageously comprises a surface sterilisation means 15 of the ice particles in the collection container 10.

The surface sterilisation means 15 advantageously comprises at least one ultraviolet germicidal lamp positioned inside the collection container 10, typically above the ice particles deposited in the collection container 10.

The detection means 12 communicates with a suitable control means 13 configured to activate the surface sterilisation means 15 only if the closed position of the hatch door 11 is detected.

The control means 13 activates the surface sterilisation means 15 according to a predefined timed program, as a function at least of the type of the ice particles produced and collected in the container 10, such as ice cubes (full or hollow), in flakes or granules, and as a function at least of the quantity of the ice particles appropriately detected in the collection container 10.

The control means 13 typically comprises a timer and a relay which control the energy supply to the surface sterilisation means 15.

The machine 1 can comprise sanitising means with ionised air and ozone 21 for sanitising internal volumes of the machine 1 which can be provided to replace or preferably in addition to the above-described sterilisation means.

Said sanitising means of ionised air and ozone 21 comprises detection means 22, control means 23 communicating with said detection means 22 and configured to activate production means of at least ionised air and ozone 24 and diffusion means 25 of ionised air and ozone.

The diffusion means 25 is advantageously configured for the diffusion of ionised air and ozone at least into the area of the forming means 31 of the ice particles in proximity of the evaporator 30 and/or at least into the container 10 of the ice particles and/or at least into the storage tank 20 for storing the drinking water in the liquid state coming from the municipal water supply and used for the formation of the ice particles.

The production means of at least ionised air and ozone 24 typically comprises an ultra-violet anti-bacterial germicidal lamp, and as well as ionised air the production means produces ozone, well known today as the most powerful existing virucide and bactericide, able to deactivate not only viruses but also a vast array of other contaminant micro-organisms possibly present in the internal volumes of the machine 1.

The control means 23 activates the production means of ionised air and ozone 24 and the diffusion means 25 according to a sequence and a predefined timed program while controlling the diffusion areas, times and the quantity of diffusion. The operation of the fully-automated monobloc machine for manufacturing ice in particles for food use according to the invention emerges clearly from the description and illustration and, in particular, is substantially as follows.

The operation is described with reference to a cycle of manufacturing of ice in particles, from an instant considered initial at which the collection container 10 is considered to be sufficiently full of ice particles, in any conformation i.e. cubes, flakes or granules.

The user accesses the collection container by opening the access hatch door 11, normally in a closed position, and manually and/or using manual tools collects the quantity of ice particles at the desired instant: this intrusion to the inside of the collection container 10, if not carried out with the due hygienic precautions, can contaminate, with micro-organisms or bacteria, the residual surface of the mass of ice particles remaining in the collection container 10.

The access hatch door 11 is closed, manually or by means of traditional closing devices of an elastic type, not illustrated in the figures.

The detection means 12 detects the closure of the access hatch door 11 and communicates this datum to the control means 13 which activates the surface sterilisation means 15, typically at least one ultraviolet germicidal anti-bacterial lamp, which illuminates the residual surface of the mass of ice particles remained in the collection container 10, according to a predefined timed program, and sterilises it.

When the volume of the ice particles remained in the collection container 10 falls below a predetermined quantity, typically a level, suitable traditional detection means, not illustrated in the figures, activates the operating means 32 which conveys the process drinking water contained in the storage tank 20 onto the forming means of the ice particles 31, where the drinking water changes from liquid to solid state, restoring the level of the ice particles produced in the collection container 10 to a predetermined value.

The activation of the production means 32, and therefore the supply thereof with the process water, reduces the level of the process water in the storage tank 20, where suitable traditional detection means, not illustrated in the figures, activates the supply of the storage tank 20 from the municipal water supply of drinking water, with traditional means, not illustrated, restoring the level to predefined quantities.

The detection means 22, on detecting the activation of the operating means 32, transfers the datum to the control means 23 which activates the production means of ionised air and ozone 24 which the diffusion means 25 advantageously transfers in known ways into the internal volumes of the machine 1 according to a sequence and a predefined timed program.

Typically, when the ice particles under formation are ice cubes (full or hollow) the diffusion means 25 transfers ionised air and ozone in the area of the forming means 31 of the ice particles in proximity of the evaporator 30 and in the container 10 of the ice particles;
when the ice particles under formation are ice in flakes or granules, the diffusion means 25 transfers ionised air and ozone into the container 10 of the ice particles and into the storage tank 20 for storing the drinking water in the liquid state.

It has been demonstrated how a fully-automated monobloc machine for manufacturing ice in particles for food use according to the invention is particularly advantageous for surface sanitising of the ice particles in the collection container, following each withdrawal of particles by the user.

A further advantage of a fully-automated monobloc machine for manufacturing ice in particles for food use according to the invention is the presence of sanitising means with ionised air and ozone 21 for sanitising internal volumes of said machine 1.

A not least advantage of a fully-automated monobloc machine for manufacturing ice in particles for food use according to the invention is that it has an automatic surface sanitising system of the ice particles in the collection container and a sanitising system of the internal volumes of the machine that are simple, effective and have an automated functioning.

A fully-automated monobloc machine for manufacturing ice in particles for food use thus conceived is susceptible to numerous modifications and variants, all falling within the scope of the inventive concept, as defined in the claims.

In practice, the materials used, as well as the dimensions, can be any according to the needs and the state of the art.

## Claims

1. A fully-automated monobloc machine (1) for manufacturing ice in particles for food use, comprising at least one storage tank (20) of process water in the liquid state, an evaporator (30) provided with forming means for forming ice particles (31) from the process water in the liquid state, a collection container (10) of the ice particles, an access hatch door (11) to said collection container (10), surface sterilisation means (15) of said ice particles present in said collection container (10), detection means (12) of the open or closed position of said access hatch door (11), and control means (13) communicating with said detection means (12) and configured to activate said surface sterilisation means (15) only if said closed position of said access hatch door is detected (11), **characterised in that** said control means (13) are configured to activate said surface sterilisation means (15) according to a predefined timed program, as a function at least of the type of the ice particles produced and collected in the container (10), and as a function at least of the quantity of the ice particles detected in the collection container (10).

2. The fully-automated monobloc machine (1) for manufacturing ice according to claim 1, **characterised in that** said ice particles are ice cubes.

3. The fully-automated monobloc machine (1) for manufacturing ice according to to claim 1, **characterised in that** said ice particles are flakes or granules of ice.

4. The fully-automated monobloc machine (1) for manufacturing ice according to one or more of the preceding claims, **characterised in that** said detection means (12) comprises a microswitch.

5. The fully-automated monobloc machine (1) for manufacturing ice according to one or more of the preceding claims, **characterised in that** said control means (13) comprises a timer and a relay.

6. The fully-automated monobloc machine (1) for manufacturing ice according to one or more of the preceding claims, **characterised in that** said surface sterilisation means (15) comprises at least one ultraviolet germicidal lamp.

7. The fully-automated monobloc machine (1) for manufacturing ice in particles for food use according to one or more of the preceding claims, **characterised in that** it comprises sanitising means with ionised air and ozone (21) for sanitising internal volumes of said machine (1).

8. The fully-automated monobloc machine (1) for manufacturing ice according to the preceding claim, **characterised in that** said sanitising means with ionised air and ozone (21) comprises detection means (22) and control means (23) communicating with said detection means (22) and configured to activate production means of ionised air and ozone (24).

9. The fully-automated monobloc machine (1) for manufacturing ice according to the preceding claim, **characterised in that** said sanitising means with ionised air and ozone (21) comprises said production means of ionised air and ozone (24) and diffusion means (25) of said ionised air and ozone.

10. The fully-automated monobloc machine (1) for manufacturing ice according to the preceding claim, **characterised in that** said diffusion means (25), when the ice particles under formation are ice in cubes, transfers said ionised air and ozone to the zone of said forming means (31) of said ice particles in proximity of said evaporator (30) and into said container (10) of said ice particles.

11. The fully-automated monobloc machine (1) for manufacturing ice according to claim 9, **characterised in that** said diffusion means (25), when the ice particles under formation are ice in flakes or granules, transfers said ionised air and ozone into said container (10) of said ice particles and into said storage tank (20) for storing the drinking water in the liquid state.

12. The fully-automated monobloc machine (1) for manufacturing ice according to any one of claims from 8 to 11, **characterised in that** said control means (23) activates said production means of ionised air and ozone (24) and said diffusion means (25) according to a sequence and a predefined timed program.

## Patentansprüche

1. Eine vollautomatische Monoblock-Maschine (1) zur Herstellung von Eispartikeln für den Lebensmittelgebrauch, umfassend mindestens einen Vorratsbehälter (20) für Prozesswasser im flüssigen Zustand, einen Verdampfer (30), der mit Formmitteln zum Formen von Eispartikeln (31) aus dem Prozesswasser im flüssigen Zustand versehen ist, einen Auffangbehälter (10) für die Eispartikel, eine Zugangsklappe (11) zu besagtem Auffangbehälter (10), Mittel zur Oberflächensterilisation (15) der in besagtem Auffangbehälter (10) vorhandenen Eispartikel, Mittel zur Erkennung (12) der geöffneten oder geschlossenen Position besagter Zugangsklappe (11) und Steuerungsmittel (13), die mit besagten Erkennungsmitteln (12) kommunizieren und konfiguriert sind, besagte Mittel zur Oberflächensterilisation (15) nur dann zu aktivieren, wenn besagte geschlossene Position der Zugangsklappe (11) erkannt wird, **dadurch gekennzeichnet, dass** besagte Steuerungsmittel (13) konfiguriert sind, besagte Mittel zur Oberflächensterilisation (15) nach einem vordefinierten Zeitprogramm zu aktivieren, in Abhängigkeit mindestens von der Art der hergestellten und im Behälter (10) gesammelten Eispartikeln und mindestens in Abhängigkeit von der Menge der im Auffangbehälter (10) erkannten Eispartikel.

2. Die vollautomatische Monoblock-Maschine (1) zur Herstellung von Eis gemäß Anspruch 1, **dadurch gekennzeichnet, dass** besagte Eispartikel Eiswürfel sind.

3. Die vollautomatische Monoblock-Maschine (1) zur Herstellung von Eis gemäß Anspruch 1, **dadurch gekennzeichnet, dass** besagte Eispartikel Eisflocken oder Eisgranulat sind.

4. Die vollautomatische Monoblock-Maschine (1) zur Herstellung von Eis gemäß einem oder mehreren der vorhergehenden Ansprüche, **dadurch gekennzeichnet, dass** besagte Erkennungsmittel (12) einen Mikroschalter umfassen.

5. Die vollautomatische Monoblock-Maschine (1) zur Herstellung von Eis gemäß einem oder mehreren der vorhergehenden Ansprüche, **dadurch gekennzeichnet, dass** besagte Steuerungsmittel (13) einen Timer und ein Relais umfassen.

6. Die vollautomatische Monoblock-Maschine (1) zur Herstellung von Eis gemäß einem oder mehreren der vorhergehenden Ansprüche, **dadurch gekennzeichnet, dass** besagte Mittel zur Oberflächensterilisation (15) mindestens eine ultraviolette keimtötende Lampe umfassen.

7. Die vollautomatische Monoblock-Maschine (1) zur Herstellung von Eispartikeln für den Lebensmittelgebrauch gemäß einem oder mehreren der vorhergehenden Ansprüche, **dadurch gekennzeichnet, dass** sie Desinfektionsmittel mit ionisierter Luft und Ozon (21) zur Desinfektion der Innenräume der Maschine (1) umfasst.

8. Die vollautomatische Monoblock-Maschine (1) zur Herstellung von Eis gemäß dem vorhergehenden Anspruch, **dadurch gekennzeichnet, dass** besagte Desinfektionsmittel mit ionisierter Luft und Ozon (21) Erkennungsmittel (22) und Steuerungsmittel (23) umfassen, die mit besagten Erkennungsmitteln (22) kommunizieren und konfiguriert sind, Produktionsmittel für ionisierte Luft und Ozon (24) zu aktivieren.

9. Die vollautomatische Monoblock-Maschine (1) zur Herstellung von Eis gemäß dem vorhergehenden Anspruch, **dadurch gekennzeichnet, dass** besagte Desinfektionsmittel mit ionisierter Luft und Ozon (21) besagte Produktionsmittel für ionisierte Luft und Ozon (24) und Verteilmittel (25) für besagte ionisierte Luft und Ozon umfassen.

10. Die vollautomatische Monoblock-Maschine (1) zur Herstellung von Eis gemäß dem vorhergehenden Anspruch, **dadurch gekennzeichnet, dass** besagte Verteilmittel (25), wenn die sich in der Bildung befindlichen Eispartikel Eiswürfel sind, besagte ionisierte Luft und Ozon in den Bereich der Formmittel (31) der Eispartikel in der Nähe des Verdampfers (30) und in den Behälter (10) der Eispartikel überführen.

11. Die vollautomatische Monoblock-Maschine (1) zur Herstellung von Eis gemäß Anspruch 9, **dadurch gekennzeichnet, dass** besagte Verteilmittel (25), wenn die sich in der Bildung befindlichen Eispartikel Eissplitter oder Eisgranulat sind, besagte ionisierte Luft und Ozon in besagten Behälter (10) der Eispartikel und in den Vorratsbehälter (20) zur Speicherung des Trinkwassers im flüssigen Zustand überführen.

12. Die vollautomatische Monoblock-Maschine (1) zur Herstellung von Eis gemäß einem der Ansprüche 8 bis 11, **dadurch gekennzeichnet, dass** besagte Steuerungsmittel (23) besagte Produktionsmittel für ionisierte Luft und Ozon (24) und besagte Verteilmittel (25) nach einer Abfolge und einem vordefinierten Zeitprogramm aktivieren.

## Revendications

1. Une machine monobloc entièrement automatisée (1) pour la fabrication de glace en particules à usage alimentaire, comprenant au moins un réservoir de stockage (20) d'eau de procédé à l'état liquide, un évaporateur (30) équipé de moyens de formation pour former des particules de glace (31) à partir de l'eau de procédé à l'état liquide, un récipient collecteur (10) des particules de glace, une porte de trappe d'accès (11) audit récipient collecteur (10), des moyens de stérilisation de surface (15) desdites particules de glace présentes dans ledit récipient collecteur (10), des moyens de détection (12) de la position ouverte ou fermée de ladite porte de trappe d'accès (11), et des moyens de contrôle (13) communiquant avec lesdits moyens de détection (12) et configurés pour activer lesdits moyens de stérilisation de surface (15) uniquement si ladite position fermée de ladite porte de trappe d'accès (11) est détectée, **caractérisée en ce que** lesdits moyens de contrôle (13) sont configurés pour activer lesdits moyens de stérilisation de surface (15) selon un programme temporel prédéfini, en fonction au moins du type des particules de glace produites et collectées dans le récipient (10), et en fonction au moins de la quantité des particules de glace détectées dans le récipient collecteur (10).

2. La machine monobloc entièrement automatisée (1) pour la fabrication de glace selon la revendication 1, **caractérisée en ce que** lesdites particules de glace sont des glaçons.

3. La machine monobloc entièrement automatisée (1) pour la fabrication de glace selon la revendication 1, **caractérisée en ce que** lesdites particules de glace sont des flocons ou des granulés de glace.

4. La machine monobloc entièrement automatisée (1) pour la fabrication de glace selon une ou plusieurs des revendications précédentes, **caractérisée en ce que** lesdits moyens de détection (12) comprennent un microswitch.

5. La machine monobloc entièrement automatisée (1) pour la fabrication de glace selon une ou plusieurs des revendications précédentes, **caractérisée en ce que** lesdits moyens de contrôle (13) comprennent un minuteur et un relais.

6. La machine monobloc entièrement automatisée (1) pour la fabrication de glace selon une ou plusieurs des revendications précédentes, **caractérisée en ce que** lesdits moyens de stérilisation de surface (15) comprennent au moins une lampe germicide ultraviolette.

7. La machine monobloc entièrement automatisée (1) pour la fabrication de glace en particules à usage alimentaire selon une ou plusieurs des revendications précédentes, **caractérisée en ce qu'**elle comprend des moyens de désinfection avec de l'air ionisé et de l'ozone (21) pour désinfecter les volumes internes de ladite machine (1).

8. La machine monobloc entièrement automatisée (1) pour la fabrication de glace selon la revendication précédente, **caractérisée en ce que** lesdits moyens de désinfection avec de l'air ionisé et de l'ozone (21) comprennent des moyens de détection (22) et des moyens de contrôle (23) communiquant avec lesdits moyens de détection (22) et configurés pour activer les moyens de production d'air ionisé et d'ozone (24).

9. La machine monobloc entièrement automatisée (1) pour la fabrication de glace selon la revendication précédente, **caractérisée en ce que** lesdits moyens de désinfection avec de l'air ionisé et de l'ozone (21) comprennent lesdits moyens de production d'air ionisé et d'ozone (24) et des moyens de diffusion (25) de l'air ionisé et de l'ozone.

10. La machine monobloc entièrement automatisée (1) pour la fabrication de glace selon la revendication précédente, **caractérisée en ce que** lesdits moyens de diffusion (25), lorsque les particules de glace en formation sont des glaçons, transfèrent l'air ionisé et l'ozone vers la zone desdits moyens de formation (31) desdites particules de glace à proximité dudit évaporateur (30) et dans ledit récipient (10) desdites particules de glace.

11. La machine monobloc entièrement automatisée (1) pour la fabrication de glace selon la revendication 9, **caractérisée en ce que** lesdits moyens de diffusion (25), lorsque les particules de glace en formation sont des flocons ou des granulés, transfèrent l'air ionisé et l'ozone dans ledit récipient (10) desdites particules de glace et dans ledit réservoir de stockage (20) pour stocker l'eau potable à l'état liquide.

12. La machine monobloc entièrement automatisée (1) pour la fabrication de glace selon l'une des revendications 8 à 11, **caractérisée en ce que** lesdits moyens de contrôle (23) activent lesdits moyens de production d'air ionisé et d'ozone (24) et lesdits moyens de diffusion (25) selon une séquence et un programme temporel prédéfini.
